(19) Europäisches Patentamt

European Patent Office

Office européen des brevets

(11) EP 1 611 790 A2

(12) EUROPEAN PATENT APPLICATION

(43) Date of publication:
04.01.2006 Bulletin 2006/01

(51) Int Cl.:
A01N 59/00 (2006.01)    A01N 59/00 (2006.01)
A01N 47/44 (2006.01)    A01N 43/80 (2006.01)
A01N 43/50 (2006.01)    A01N 37/34 (2006.01)
A01N 35/02 (2006.01)    A01N 33/18 (2006.01)
A01N 33/06 (2006.01)

(21) Application number: 05253814.7

(22) Date of filing: 20.06.2005

(84) Designated Contracting States:
AT BE BG CH CY CZ DE DK EE ES FI FR GB GR
HU IE IS IT LI LT LU MC NL PL PT RO SE SI SK TR
Designated Extension States:
AL BA HR LV MK YU

(30) Priority: 02.07.2004 US 585183 P

(71) Applicant: ROHM AND HAAS COMPANY
Philadelphia,
Pennsylvania 19106-2399 (US)

(72) Inventor: Williams, Terry Michael
Ambler
Pennsylvania 19002 (US)

(74) Representative: Kent, Venetia Katherine
Rohm and Haas (UK) Ltd
European Patent Department
28th. Floor, City Point
One Ropemaker Street
London EC2Y 9HS (GB)

(54) **Microbicidal composition**

(57)    A microbicidal composition containing: (a) a stabilized hypochlorite and bromide composition; and (b) at least one microbicide selected from among 2-methyl-4-isothiazolin-3-one, 5-choloro-2-methyl-4-isothiazolin-3-one, 4,5-dichloro-2-N-octyl-4-isothiazolin-3-one, 1,2-benzisothiazolin-3-one, 3-bromo-1-chloro-5,5-dimethylhydantoin, poly(hexamethylenebiguanide hydrochloride), alkyl-dimethyl-benzylammonium chloride, pentane-1,5-dial (glutaraldehyde), 5-bromo-2-nitro-1,3-propane-diol and 2,2-dibromo-3-nitrilopropionamide.

**Description**

This invention relates to a synergistic combination of selected microbicides having greater activity than would be observed for the individual microbicides.

In some cases, commercial microbicides cannot provide effective control of microorganisms, even at high use concentrations, due to weak activity against certain types of microorganisms, e.g., those resistant to some microbicides, or due to aggressive environmental conditions. Combinations of different microbicides are sometimes used to provide overall control of microorganisms in a particular end use environment. For example, a combination of (i) a mixture of 5-chloro-2-methylisothiazolin-3-one and 2-methylisothiazolin-3-one; and (ii) stabilized sodium hypobromite, is disclosed in U.S. Pat. No. 6,322,749. However, there is a need for additional combinations of microbicides having enhanced activity against various strains of microorganisms to provide effective control of the microorganisms.. Moreover, there is a need for combinations containing lower levels of individual microbicides for environmental and economic benefit. The problem addressed by this invention is to provide such additional combinations of microbicides.

STATEMENT OF THE INVENTION

The present invention is directed to a microbicidal composition comprising: (a) a stabilized hypochlorite and bromide composition; and (b) at least one microbicide selected from among 2-methyl-4-isothiazolin-3-one, 5-choloro-2-methyl-4-isothiazolin-3-one, 4,5-dichloro-2-N-octyl-4-isothiazolin-3-one, 1,2-benzisothiazolin-3-one, 3-bromo-1-chloro-5,5-dimethylhydantoin, poly(hexamethylenebiguanide hydrochloride), alkyl-dimethyl-benzylammonium chloride, pentane-1,5-dial (glutaraldehyde), 5-bromo-2-nitro-1,3-propane-diol and 2,2-dibromo-3-nitrilopropionamide.

The present invention is further directed to a method for treating water by adding: (a) a stabilized hypochlorite and bromide composition; and (b) at least one microbicide selected from among 2-methyl-4-isothiazolin-3-one, 5-choloro-2-methyl-4-isothiazolin-3-one, 4,5-dichloro-2-N-octyl-4-isothiazolin-3-one, 1,2-benzisothiazolin-3-one, 3-bromo-1-chloro-5,5-dimethylhydantoin, poly(hexamethylenebiguanide hydrochloride), alkyl-dimethyl-benzylammonium chloride, pentane-1,5-dial (glutaraldehyde), 5-bromo-2-nitro-1,3-propane-diol and 2,2-dibromo-3-nitrilopropionamide.

DETAILED DESCRIPTION OF THE INVENTION

"MI" is 2-methyl-4-isothiazolin-3-one, also referred to by the name 2-methyl-3-isothiazolone. "CMI" is 5-chloro-2-methyl-4-isothiazolin-3-one. "DCOIT" is 4,5-dichloro-2-N-octyl-4-isothiazolin-3-one. "BIT" is 1,2-benzisothiazolin-3-one. "BCDMH" is 3-bromo-1-chloro-5,5-dimethylhydantoin. "PHMB" is poly(hexamethylenebiguanide hydrochloride). "ADBAC" is alkyl-dimethyl-benzylammonium chloride in which alkyl groups can be present with various ratios of $C_{12}$-$C_{18}$. "BNPD" is 5-bromo-2-nitro-1,3-propane-diol. "DBNPA" is 2,2-dibromo-3-nitrilopropionamide. "GLUT" is pentane-1,5-dial (glutaraldehyde).

A "stabilized hypochlorite and bromide composition" is a composition comprising an alkali or alkaline earth metal hypochlorite, a bromide ion source and a stabilizer. Preferably, the stabilizer is sulfamic acid or an alkali metal sulfamate. Examples of stabilized hypochlorite and bromide compositions are disclosed in U.S. Pat. Nos. 6,478,972 and 6,533,958, in which the hypochlorite is first stabilized with sulfamic acid at elevated pH (>11). Then a soluble bromide source, preferable sodium bromide, is added to the mix. Under these conditions, the stabilized hypochlorite and bromide do not react at concentrated levels at high pH. When diluted at use concentrations (0.05-10 ppm total residual oxidant reported as $Cl_2$), the stabilized hypochlorite oxidizes the bromide to form free residual oxidant. In one embodiment of the invention, the composition contains from 1% to 15% alkali or alkaline earth metal hypochlorite, from 0.1% to 10% bromide ion source, and from 1% to 24% alkali metal sulfamate. In one embodiment of the invention, the composition contains from 5% to 10% sodium hypochlorite, from 0.5% to 6% sodium bromide, and from 5% to 15% sodium sulfamate. In one embodiment of the invention, the pH of the stabilized hypochlorite and bromide composition is at least 5, more preferably at least 10, and most preferably at least 12; preferably, the pH is no more than 13.5. In liquid formulations, preferably the pH is at least 10.

As used herein, the following terms have the designated definitions, unless the context clearly indicates otherwise. The term "microbicide" refers to a compound capable of inhibiting the growth of or controlling the growth of microorganisms at a locus; microbicides include bactericides, fungicides and algaecides. The term "microorganism" includes, for example, fungi (such as yeast and mold), bacteria and algae. The term "locus" refers to an industrial system or product subject to contamination by microorganisms. The following abbreviations are used throughout the specification: ppm = parts per million by weight (weight/weight), mL = milliliter, ATCC = American Type Culture Collection, MBC = minimum biocidal concentration, and MIC = minimum inhibitory concentration. Unless otherwise specified, temperatures are in degrees centigrade (°C), and references to percentages (%) are by weight. Amounts of stabilized hypochlorite and bromide composition or BCDMH used in determination of weight ratios are in ppm of total oxidant, reported as $Cl_2$, using the standard DPD (N,N-diethyl-p-phenylenediamine) colorimetric test method (available from Hach Chemical Co.,

Loveland CO). Amounts of organic microbicides other than BCDMH are given on an active ingredient basis in ppm.

The compositions of the present invention unexpectedly have been found to provide enhanced microbicidal efficacy at a combined active ingredient level lower than that of the individual microbicides. In one embodiment of the invention, those antimicrobial compositions which contain halogenated 3-isothiazolones contain relatively low levels thereof, preferably no more than 1000 ppm, more preferably no more than 500 ppm, more preferably no more than 100 ppm, and most preferably no more than 50 ppm. Concentrations of halogenated 3-isothiazolones in the composition of this invention are based on the total weight of active ingredients in the composition, i.e., the microbicides exclusive of any amounts of solvents, carriers, dispersants, stabilizers or other materials which may be present.

In one embodiment of the invention, the antimicrobial composition comprises a stabilized hypochlorite and bromide composition; 2-methyl-4-isothiazolin-3-one and 5-chloro-2-methyl-4-isothiazolin-3-one. The 2-methyl-4-isothiazolin-3-one and the 5-chloro-2-methyl-4-isothiazolin-3-one may be present in any ratio. A preferred composition contains 5-chloro-2-methyl-4-isothiazolin-3-one and 2-methyl-4-isothiazolin-3-one in a ratio up to 4:1; a commercial product contains these compounds in a ratio of about 3:1. In one preferred embodiment, the ratio of 5-chloro-2-methyl-4-isothiazolin-3-one to 2-methyl-4-isothiazolin-3-one is from 4:1 to 1:1. Preferably, a weight ratio of stabilized hypochlorite and bromide composition to a mixture of 5-chloro-2-methyl-4-isothiazolin-3-one and 2-methyl-4-isothiazolin-3-one is from 1:20 to 300:1, more preferably from 1:5 to 300:1, and most preferably from 1:5 to 270:1. In one preferred embodiment of the invention, the weight ratio is from 1:5 to 10:1. Preferably, the use concentration of the stabilized hypochlorite and bromide composition ranges from about 0.05-20 ppm total oxidant and the mixture of 5-chloro-2-methyl-4-isothiazolin-3-one and 2-methyl-4-isothiazolin-3-one ranges from about 0.01-50 ppm. Most preferably, the use concentration of the stabilized hypochlorite and bromide composition ranges from about 1-10 ppm total oxidant and the mixture of 5-chloro-2-methyl-4-isothiazolin-3-one and 2-methyl-4-isothiazolin-3-one ranges from about 0.5-25 ppm.

In one embodiment of the invention, the antimicrobial composition comprises a stabilized hypochlorite and bromide composition and 4,5-dichloro-2-n-octyl-4-isothiazolin-3-one. Preferably, a weight ratio of stabilized hypochlorite and bromide composition to 4,5-dichloro-2-n-octyl-4-isothiazolin-3-one is from 1:100 to 100:1, more preferably from 1:75 to 75:1, and most preferably from 1:71 to 68:1. In one preferred embodiment, the weight ratio is from 1:6 to 6:1. Preferably, the use concentration of the stabilized hypochlorite and bromide composition ranges from about 0.05-20 ppm total oxidant and 4,5-dichloro-2-n-octyl-4-isothiazolin-3-one ranges from about 0.01-20 ppm. Most preferably, the use concentration of the stabilized hypochlorite and bromide composition ranges from about 1-10 ppm total oxidant and 4,5-dichloro-2-n-octyl-4-isothiazolin-3-one ranges from about 0.5- 10 ppm.

In one embodiment of the invention, the antimicrobial composition comprises a stabilized hypochlorite and bromide composition and 1,2-benzisothiazolin-3-one. Preferably, a weight ratio of stabilized hypochlorite and bromide composition to 1,2-benzisothiazolin-3-one is from 1:400 to 50:1, more preferably from 1:375 to 40:1, and most preferably from 1:357 to 33:1. In one preferred embodiment, the weight ratio is from 1:50 to 2:1. Preferably, the use concentration of the stabilized hypochlorite and bromide composition ranges from about 0.05-20 ppm total oxidant and 1,2-benzisothiazolin-3-one ranges from about 0.1-500 ppm. Most preferably, the use concentration of the stabilized hypochlorite and bromide composition ranges from about 1-10 ppm total oxidant and 1,2-benzisothiazolin-3-one ranges from about 10-300 ppm.

In one embodiment of the invention, the antimicrobial composition comprises a stabilized hypochlorite and bromide composition and 3-bromo-1-chloro-5,5-dimethylhydantoin. Preferably, a weight ratio of stabilized hypochlorite and bromide composition to 3-bromo-1-chloro-5,5-dimethylhydantoin is from 1:30 to 120:1, more preferably from 1:25 to 115:1, and most preferably from 1:24 to 113:1. In one preferred embodiment, the weight ratio is from 1:10 to 10:1. Preferably, the use concentration of the stabilized hypochlorite and bromide composition ranges from about 0.05-20 ppm total oxidant and 3-bromo-1-chloro-5,5-dimethylhydantoin ranges from about 0.05-100 ppm total oxidant. Most preferably, the use concentration of the stabilized hypochlorite and bromide composition ranges from about 1-10 ppm total oxidant and 3-bromo-1-chloro-5,5-dimethylhydantoin ranges from about 0.5-10 ppm total oxidant.

In one embodiment of the invention, the antimicrobial composition comprises a stabilized hypochlorite and bromide composition and poly(hexamethylenebiguanide hydrochloride). Preferably, a weight ratio of stabilized hypochlorite and bromide composition to poly(hexamethylenebiguanide hydrochloride) is from 1:100 to 150:1, more preferably from 1:50 to 140:1, and most preferably from 1:45 to 134:1. In one preferred embodiment, the weight ratio is from 1:20 to 10:1. Preferably, the use concentration of the stabilized hypochlorite and bromide composition ranges from about 0.05-20 ppm total oxidant and poly(hexamethylenebiguanide hydrochloride) ranges from about 0.01-200 ppm. Most preferably, the use concentration of the stabilized hypochlorite and bromide composition ranges from about 1-10 ppm total oxidant and poly(hexamethylenebiguanide hydrochloride) ranges from about 1-10 ppm.

In one embodiment of the invention, the antimicrobial composition comprises a stabilized hypochlorite and bromide composition and alkyl-dimethyl-benzylammonium chloride. Preferably, a weight ratio of stabilized hypochlorite and bromide composition to alkyl-dimethyl-benzylammonium chloride is from 1:200 to 5:1, more preferably from 1:200 to 3:1, and most preferably from 1:192 to 2.6:1. In one preferred embodiment, the weight ratio is from 1:10 to 2:1. Preferably, the use concentration of the stabilized hypochlorite and bromide composition ranges from about 0.05-20 ppm total oxidant and alkyl-dimethyl-benzylammonium chloride ranges from about 0.1-200 ppm. Most preferably, the use concentration of the stabilized hypochlorite and bromide composition ranges from about 1-10 ppm total oxidant and alkyl-dimethyl-benzylammonium chloride ranges from about 5-25 ppm.

In one embodiment of the invention, the antimicrobial composition comprises a stabilized hypochlorite and bromide composition and pentane-1,5-dial. Preferably, a weight ratio of stabilized hypochlorite and bromide composition to pentane-1,5-dial is from 1:200 to 1:5, more preferably from 1:200 to 1:10, and most preferably from 1:192 to 1:12. In one preferred embodiment, the weight ratio is from 1:100 to 1:20. Preferably, the use concentration of the stabilized hypochlorite and bromide composition ranges from about 0.05-20 ppm total oxidant and pentane-1,5-dial ranges from about 5-500 ppm. Most preferably, the use concentration of the stabilized hypochlorite and bromide composition ranges from about 1-10 ppm total oxidant and pentane-1,5-dial ranges from about 15-200 ppm.

In one embodiment of the invention, the antimicrobial composition comprises a stabilized hypochlorite and bromide composition and 5-bromo-2-nitro-1,3-propane-diol. Preferably, a weight ratio of stabilized hypochlorite and bromide composition to 5-bromo-2-nitro-1,3-propane-diol is from 1:20 to 70:1, preferably from 1:5 to 70:1, and most preferably from 1.3:1 to 68:1. In one preferred embodiment, the weight ratio is from 1:1 to 68:1. Preferably, the use concentration of the stabilized hypochlorite and bromide composition ranges from about 0.05-20 ppm total oxidant and 5-bromo-2-nitro-1,3-propane-diol ranges from about 0.1-500 ppm. Most preferably, the use concentration of the stabilized hypochlorite and bromide composition ranges from about 1-10 ppm total oxidant and 5-bromo-2-nitro-1,3-propane-diol ranges from about 5-200 ppm.

In one embodiment of the invention, the antimicrobial composition comprises a stabilized hypochlorite and bromide composition and 2,2-dibromo-3-nitrilopropionamide. Preferably, a weight ratio of stabilized hypochlorite and bromide composition to 2,2-dibromo-3-nitrilopropionamide is from 1:50 to 2:1, more preferably from 1:40 to 1.5:1, and most preferably from 1:31 to 1.3:1. In one preferred embodiment, the weight ratio is from 1:10 to 1:1. Preferably, the use concentration of the stabilized hypochlorite and bromide composition ranges from about 0.05-20 ppm total oxidant and 2,2-dibromo-3-nitrilopropionamide ranges from about 0.1-200 ppm. Most preferably, the use concentration of the stabilized hypochlorite and bromide composition ranges from about 1-10 ppm total oxidant and 2,2-dibromo-3-nitrilo-propionamide ranges from about 2-25 ppm.

The microbicides in the composition of this invention may be used "as is" or may first be formulated with a solvent or a solid carrier. Suitable solvents include, for example, water; glycols, such as ethylene glycol, propylene glycol, diethylene glycol, dipropylene glycol, polyethylene glycol, and polypropylene glycol; glycol ethers; alcohols, such as methanol, ethanol, propanol, phenethyl alcohol and phenoxypropanol; ketones, such as acetone and methyl ethyl ketone; esters, such as ethyl acetate, butyl acetate, triacetyl citrate, and glycerol triacetate; carbonates, such as propylene carbonate and dimethyl carbonate; and mixtures thereof. It is preferred that the solvent is selected from water, glycols, glycol ethers, esters and mixtures thereof. Suitable solid carriers include, for example, cyclodextrin, silicas, diatomaceous earth, waxes, cellulosic materials, alkali and alkaline earth (e.g., sodium, magnesium, potassium) metal salts (e.g., chloride, nitrate, bromide, sulfate) and charcoal.

When a microbicide component is formulated in a solvent, the formulation may optionally contain surfactants. When such formulations contain surfactants, they are generally in the form of emulsive concentrates, emulsions, microemulsive concentrates, or microemulsions. Emulsive concentrates form emulsions upon the addition of a sufficient amount of water. Microemulsive concentrates form microemulsions upon the addition of a sufficient amount of water. Such emulsive and microemulsive concentrates are generally well known in the art; it is preferred that such formulations are free of surfactants. U.S. Patent No. 5,444,078 may be consulted for further general and specific details on the preparation of various microemulsions and microemulsive concentrates.

A microbicide component also can be formulated in the form of a dispersion. The solvent component of the dispersion can be an organic solvent or water, preferably water. Such dispersions can contain adjuvants, for example, co-solvents, thickeners, anti-freeze agents, dispersants, fillers, pigments, surfactants, biodispersants, sulfosuccinates, terpenes, furanones, polycations, stabilizers, scale inhibitors and anti-corrosion additives.

The microbicides are preferred to be formulated separately, due to the high reactivity and high pH of the stabilized hypochlorite component. However, solid compositions may allow both components to be formulated together thus eliminating the undesired cross reactivity. When both microbicides are each first formulated with a solvent, the solvent used for the first microbicide may be the same as or different from the solvent used to formulate the other commercial microbicide, although water is preferred for most industrial biocide applications. It is preferred that the two solvents are miscible.

Those skilled in the art will recognize that the microbicide components of the present invention may be added to a locus sequentially, simultaneously, or may be combined before being added to the locus. It is preferred that the first microbicide and the second microbicide component be added to a locus simultaneously or sequentially. When the microbicides are added simultaneously or sequentially, each may individual components may contain adjuvants, such as, for example, solvent, thickeners, anti-freeze agents, colorants, sequestrants (such as ethylenediamine-tetraacetic acid, ethylenediaminedisuccinic acid, iminodisuccinic acid and salts thereof), dispersants, surfactants, biodispersants, sulfosuccinates, terpenes, furanones, polycations, stabilizers, scale inhibitors and anti-corrosion additives.

The microbicidal compositions of the present invention can be used to inhibit the growth of microorganisms or higher forms of aquatic life (such as protozoans, invertebrates, bryozoans, dinoflagellates, crustaceans, mollusks, etc) by introducing a microbicidally effective amount of the compositions onto, into, or at a locus subject to microbial attack. Suitable loci include, for example: industrial process water; electrocoat deposition systems,; cooling towers; air washers; gas scrubbers; mineral slurries; wastewater treatment; ornamental fountains; reverse osmosis filtration; ultrafiltration; ballast water; evaporative condensers; heat exchangers; pulp and paper processing fluids and additives; starch; plastics; emulsions; dispersions; paints; latices; coatings, such as varnishes; construction products, such as mastics, caulks, and sealants; construction adhesives, such as ceramic adhesives, carpet backing adhesives, and laminating adhesives; industrial or consumer adhesives; photographic chemicals; printing fluids; household products, such as bathroom and kitchen cleaners; cosmetics; toiletries; shampoos; soaps; detergents; industrial cleaners; floor polishes; laundry rinse water; metalworking fluids; conveyor lubricants; hydraulic fluids; leather and leather products; textiles; textile products; wood and wood products, such as plywood, chipboard, flakeboard, laminated beams, oriented strandboard, hardboard, and particleboard; petroleum processing fluids; fuel; oilfield fluids, such as injection water, fracture fluids, and drilling muds; agriculture adjuvant preservation; surfactant preservation; medical devices; diagnostic reagent preservation; food preservation, such as plastic or paper food wrap; food, beverage, and industrial process pasteurizers; toilet bowls; recreational water; pools; and spas.

Preferably, the microbicidal compositions of the present invention are used to inhibit the growth of microorganisms at a locus selected from one or more of industrial process water, cooling towers; air washers; mineral slurries; wastewater treatment; ornamental fountains; reverse osmosis filtration; ultrafiltration; ballast water; evaporative condensers; heat exchangers; pulp and paper processing fluids and additives; food, beverage, and industrial process pasteurizers; toilet bowls; pools; and spas. In particular, the microbicidal compositions are useful in cooling water; pulp and paper processing fluids and additives; ornamental fountains; and ballast water.

The specific amount of the composition of this invention necessary to inhibit or control the growth of microorganisms and higher aquatic life forms in a locus depends upon the particular locus to be protected. Typically, the amount of the composition of the present invention to control the growth of microorganisms in a locus is sufficient if it provides from 0.1 to 1,000 ppm active ingredient of the composition in the locus. It is preferred that the active ingredients of the composition be present in the locus in an amount of at least 0.5 ppm, more preferably at least 4 ppm and most preferably at least 10 ppm. It is preferred that the active ingredients of the composition be present in the locus in an amount of no more than 1000 ppm, more preferably no more than 500 ppm, and most preferably no more than 200 ppm.

EXAMPLES

Compounds Tested

Stabilized hypochlorite and bromide aqueous solution (7.1 % sodium hypochlorite + 1.5 % sodium bromide + 11.8% sulfamic acid); CMIT/MIT (5-Chloro-2-methyl-4-isothiazolin-3-one + 2-Methyl-4-isothiazolin-3-one); DCOIT (4,5-Dichloro-2-n-octyl-4-isothiazolin-3-one); BIT (1,2-Benzisothiazolin-3-one); BCDMH (3-Bromo-1-chloro-5,5-dimethylhydantoin); PHMB (Poly(hexamethylenebiguanide hydrochloride)); ADBAC (Alkyl*dimethyl benzyl ammonium chloride, *50%$C_{14}$, 40%$C_{12}$, 10%$C_{16}$); GLUT (Pentane-1,5-dial; glutaraldehyde); and DBNPA (2,2-Dibromo-nitrilo-propionamide); BNPD (5'Bromo'2-nitro- 1,3'propandiol).

Materials and Methods

The synergism of the combination of the present invention was demonstrated by testing a wide range of concentrations and ratios of the compounds.

One measure of synergism is the industrially accepted method described by Kull, F.C.; Eisman, P.C.; Sylwestrowicz, H.D. and Mayer, R.L., in Applied Microbiology 9:538-541 (1961), using the ratio determined by the formula:

$$Q_a/Q_A + Q_b/Q_B = \text{Synergy Index (''SI'')}$$

wherein:

$Q_A$ = concentration of compound A (first component) in ppm, acting alone, which produced an end point (MIC or MBC of Compound A).

$Q_a$ =    concentration of compound A in ppm, in the mixture, which produced an end point.

$Q_B$ =    concentration of compound B (second component) in ppm, acting alone, which produced an end point (MIC or MBC of Compound B).

$Q_b$ =    concentration of compound B in ppm, in the mixture, which produced an end point.

When the sum of $Q_a/Q_A$ and $Q_b/Q_B$ is greater than one, antagonism is indicated. When the sum is equal to one, additivity is indicated, and when less than one, synergism is demonstrated. The lower the SI, the greater the synergy shown by that particular mixture. The minimum inhibitory concentration (MIC) of a microbicide is the lowest concentration tested under a specific set of conditions that prevents the growth of added microorganisms.

The tests were set up with 96-well microtiter plate using the Biomek™ 2000 Workstation employing a high resolution MIC/MBC combination test method, which provides multiple combinations of two biocides in one test plate. The lowest concentration of the biocide where no growth (inhibition) was observed in the mineral salts plus glucose medium was defined as the minimum inhibitory concentrations (MIC) of the combination. Samples (10 μl) taken from each well of the MIC plate were transferred to fresh media in the MBC plates and observed for the presence of surviving (viable) microorganisms over time. No growth in the wells of the MBC plates indicated the concentration of biocides which provided effective killing ($<10^2$ /ml) of the microorganisms from the MIC plates. The lowest level of the biocides which showed no growth in the MBC plates was defined as the minimum biocidal concentration (MBC). The synergistic ratio range of the biocide combination is a reflection of the total activity of both inhibition (MIC) and killing MBC).

Tests were done using a mineral salts medium with 2% glucose (pH 7.4) for the MIC tests and Trypticase Soy Broth (TSB; pH 7.2) plus 24 ppm thioglycolate for recovery of viable cells in the MBC tests. Each well received 20 μl of a 24-hour pure culture suspension of *Pseudomonas aeruginosa* (ATCC 15442) at a final density of $10^6$ colony forming units (CFU) per ml. Cell density was adjusted to an optical density of 0.09 at 660 nm using a spectrophotometer.

MIC plates were incubated at 30°C for 48 hours and were read both using the microtiter plate reader (Optical Density of 0.05 nm) and visually for the presence or absence of microbial growth. MBC evaluations were made after several days incubation at 30°C. Tests were set up in duplicate with different starting concentrations of biocides to increase the number of possible combinations displaying antimicrobial activity.

The test results for demonstration of synergy of the microbicide combinations of the present invention are shown below in the Tables. Data presented in Tables is the day of observation, tests number and type (ex., MIC 1 = MIC test one vs MBC 2 = MBC test two), concentrations of biocides showing activity alone and in combination, synergy indices, and ratios of the stabilized hypochlorite and bromide biocide: test biocide.

All biocide results are reported on an active ingredient basis in ppm (parts per million). The stabilized hypochlorite and bromide biocide and BCDMH biocides were dosed according to ppm as total oxidant (reported as $Cl_2$).

RESULTS

Synergy data for stabilized hypochlorite + sodium bromide in combination with the following biocides are shown in Tables (1-9), respectively.

CMIT/MIT

DCOIT

BIT

BCDMH

PHMB

ADBAC

GLUT

BNPD and

DBNPA

EP 1 611 790 A2

**Summary of Synergy Data**

| Stabilized Hypochlorite-Sodium Bromide in Combination with the Following Biocides | Synergy Index Values | Synergy Ratios<br><br>Stabilized Hypochlorite and bromide : Biocide "A" |
|---|---|---|
| **Isothiazolones** | | |
| CMIT/MIT | 0.20 – 0.88 | 1:5 to 270:1 |
| DCOIT | 0.17 – 0.94 | 1:71 to 68:1 |
| BIT | 0.13 – 0.95 | 1:357 to 33:1 |
| **Cationics** | | |
| PHMB | 0.19 – 0.95 | 1:45 to 134:1 |
| ADBAC | 0.53 – 0.75 | 1:192 to 3:1 |
| **Aldehydes** | | |
| GLUT | 0.54 – 0.75 | 1:192 to 1:12 |
| **Organo-Bromines** | | |
| BNPD | 0.50 – 0.92 | 1:1 to 68:1 |
| DBNPA | 0.34 – 0.75 | 1:31 to 1.3:1 |
| **Chlorine-Bromine Oxidizers** | 0.21 – 0.95 | 1:24 to 113:1 |
| BCDMH | | |

**Table 1. Combination of CMIT/MIT(A) and Stabilized Hypochlorite and Bromide (B)**

| Day | Expt | A alone | B alone | a comb. | b comb. | a/A | b/B | SI | Ratio of Biocide b:a |
|---|---|---|---|---|---|---|---|---|---|
| 1 | MIC 1 | 0.31 | 2.70 | 0.18 | 0.50 | 0.58 | 0.19 | 0.77 | 2.8:1 |
| | | 0.31 | 2.70 | 0.18 | 0.26 | 0.58 | 0.10 | 0.68 | 1.4:1 |
| | | 0.31 | 2.70 | 0.08 | 0.50 | 0.26 | 0.19 | 0.44 | 6.3:1 |
| | | 0.31 | 2.70 | 0.04 | 0.50 | 0.13 | 0.19 | 0.31 | 12:1 |
| | | 0.31 | 2.70 | 0.02 | 0.50 | 0.06 | 0.19 | 0.25 | 25:1 |
| | | 0.31 | 2.70 | 0.01 | 0.50 | 0.03 | 0.19 | 0.22 | 50:1 |
| 1 | MBC 1 | 0.31 | 5.40 | 0.18 | 1.35 | 0.58 | 0.25 | 0.83 | 7.5:1 |
| | | 0.31 | 5.40 | 0.08 | 2.70 | 0.26 | 0.50 | 0.76 | 34:1 |
| | | 0.31 | 5.40 | 0.08 | 1.35 | 0.26 | 0.25 | 0.51 | 17:1 |
| | | 0.31 | 5.40 | 0.04 | 2.70 | 0.13 | 0.50 | 0.63 | 68:1 |
| | | 0.31 | 5.40 | 0.04 | 1.35 | 0.13 | 0.25 | 0.38 | 34:1 |
| | | 0.31 | 5.40 | 0.02 | 2.70 | 0.06 | 0.50 | 0.56 | 135:1 |
| | | 0.31 | 5.40 | 0.01 | 2.70 | 0.03 | 0.50 | 0.53 | 270:1 |
| | | 1.25 | 2.03 | 0.63 | 0.51 | 0.50 | 0.25 | 0.76 | 1:1.2 |
| | | 1.25 | 2.03 | 0.31 | 1.01 | 0.25 | 0.50 | 0.75 | 3.3:1 |
| 1 | MBC 2 | 1.25 | 2.70 | 0.63 | 0.26 | 0.50 | 0.10 | 0.60 | 1:2.4 |
| | | 1.25 | 2.70 | 0.63 | 0.13 | 0.50 | 0.05 | 0.55 | 1:4.9 |
| | | 1.25 | 2.70 | 0.31 | 0.50 | 0.25 | 0.19 | 0.43 | 1.6:1 |
| | | 1.25 | 2.70 | 0.31 | 0.26 | 0.25 | 0.10 | 0.34 | 1:1.2 |
| | | 1.25 | 2.70 | 0.18 | 0.50 | 0.14 | 0.19 | 0.33 | 2.8:1 |

7

Biocide concentrations for stabilized hypochlorite and bromide are reported as ppm total oxidant and CMIT/MIT concentrations are reported as ppm active ingredient.

Table 1 cont. CMIT/MIT(A) and Stabilized Hypochlorite and Bromide (B)

| Day | Expt | A alone | B alone | a comb. | b comb. | a/A | b/B | SI | Ratio of Biocide b:a |
|---|---|---|---|---|---|---|---|---|---|
| 3 | MIC 1 | 0.31 | 2.70 | 0.08 | 1.35 | 0.26 | 0.50 | 0.76 | 19:1 |
| | | 0.31 | 2.70 | 0.04 | 1.35 | 0.13 | 0.50 | 0.63 | 34:1 |
| | | 1.25 | 2.03 | 0.63 | 0.51 | 0.50 | 0.25 | 0.76 | 1:1.2 |
| | | 1.25 | 2.03 | 0.31 | 1.01 | 0.25 | 0.50 | 0.75 | 3.3:1 |
| | | 1.25 | 2.70 | 0.63 | 0.50 | 0.50 | 0.19 | 0.69 | 1:1.3 |
| | | 1.25 | 2.70 | 0.63 | 0.26 | 0.50 | 0.10 | 0.60 | 1:2.4 |
| | | 1.25 | 2.70 | 0.31 | 0.50 | 0.25 | 0.19 | 0.43 | 1.6:1 |
| | | 1.25 | 2.70 | 0.18 | 0.50 | 0.14 | 0.19 | 0.33 | 2.8:1 |
| | | 1.25 | 2.70 | 0.04 | 0.50 | 0.03 | 0.19 | 0.22 | 13:1 |
| 3 | MIC 2 | 1.25 | 2.70 | 0.63 | 0.50 | 0.50 | 0.19 | 0.69 | 1:1.3 |
| | | 1.25 | 2.70 | 0.63 | 0.26 | 0.50 | 0.10 | 0.60 | 1:2.4 |
| | | 1.25 | 2.70 | 0.63 | 0.13 | 0.50 | 0.05 | 0.55 | 1:4.9 |
| | | 1.25 | 2.70 | 0.31 | 0.50 | 0.25 | 0.19 | 0.43 | 1.6:1 |
| | | 1.25 | 2.70 | 0.31 | 0.26 | 0.25 | 0.10 | 0.34 | 1:1.2 |
| | | 1.25 | 2.70 | 0.18 | 0.50 | 0.14 | 0.19 | 0.33 | 2.8:1 |
| | | 1.25 | 2.70 | 0.04 | 0.50 | 0.03 | 0.19 | 0.22 | 13:1 |
| | | 1.25 | 2.70 | 0.02 | 0.50 | 0.02 | 0.19 | 0.20 | 25:1 |
| | | 1.25 | 2.03 | 0.63 | 0.51 | 0.50 | 0.25 | 0.76 | 1:1.2 |
| | | 0.31 | 2.70 | 0.08 | 1.35 | 0.26 | 0.50 | 0.76 | 17:1 |

Biocide concentrations for stabilized hypochlorite and bromide are reported as ppm total oxidant and CMIT/MIT concentrations are reported as ppm active ingredient.

Table 1 cont. CMIT/MIT(A) and Stabilized Hypochlorite and Bromide (B)

| Day | Expt | A alone | B alone | a comb. | b comb. | a/A | b/B | SI | Ratio of Biocide b:a |
|---|---|---|---|---|---|---|---|---|---|
| 3 | MBC 1 | 0.31 | 2.70 | 0.08 | 1.35 | 0.26 | 0.50 | 0.76 | 17:1 |
| | | 0.31 | 2.70 | 0.04 | 1.35 | 0.13 | 0.50 | 0.63 | 34:1 |
| | | 1.25 | 2.03 | 0.63 | 0.51 | 0.50 | 0.25 | 0.76 | 1:1.2 |
| | | 1.25 | 2.03 | 0.31 | 1.01 | 0.25 | 0.50 | 0.75 | 3.3:1 |
| | | 1.25 | 4.05 | 0.18 | 2.03 | 0.14 | 0.50 | 0.65 | 11:1 |
| | | 1.25 | 4.05 | 0.08 | 2.03 | 0.06 | 0.50 | 0.57 | 25:1 |
| | | 1.25 | 4.05 | 0.04 | 2.03 | 0.03 | 0.50 | 0.53 | 51:1 |
| | | 1.25 | 4.05 | 0.02 | 2.03 | 0.02 | 0.50 | 0.52 | 102:1 |
| | | 1.25 | 4.05 | 0.01 | 2.03 | 0.01 | 0.50 | 0.51 | 203:1 |
| 3 | MBC 2 | 5.00 | 5.40 | 2.50 | 2.03 | 0.50 | 0.38 | 0.88 | 1:1.2 |
| | | 5.00 | 5.40 | 2.50 | 1.01 | 0.50 | 0.19 | 0.69 | 1:2.5 |
| | | 5.00 | 5.40 | 1.25 | 2.03 | 0.25 | 0.38 | 0.63 | 1.6:1 |
| | | 5.00 | 5.40 | 1.25 | 1.01 | 0.25 | 0.19 | 0.44 | 1:1.2 |
| | | 5.00 | 5.40 | 1.25 | 0.51 | 0.25 | 0.09 | 0.34 | 1:2.5 |
| | | 5.00 | 5.40 | 0.63 | 4.50 | 0.13 | 0.83 | 0.96 | 7.1:1 |
| | | 5.00 | 5.40 | 0.63 | 2.03 | 0.13 | 0.38 | 0.50 | 3.2:1 |
| | | 5.00 | 5.40 | 0.63 | 1.01 | 0.13 | 0.19 | 0.31 | 1.6:1 |
| | | 5.00 | 5.40 | 0.63 | 0.51 | 0.13 | 0.09 | 0.22 | 1:1.2 |
| | | 5.00 | 5.40 | 0.31 | 2.03 | 0.06 | 0.38 | 0.44 | 6.6:1 |
| | | 5.00 | 5.40 | 0.18 | 4.50 | 0.04 | 0.83 | 0.87 | 25:1 |
| | | 5.00 | 5.40 | 0.18 | 2.03 | 0.04 | 0.38 | 0.41 | 11:1 |

Table continued

| Day | Expt | A alone | B alone | a comb. | b comb. | a/A | b/B | SI | Ratio of Biocide b:a |
|---|---|---|---|---|---|---|---|---|---|
|  |  | 5.00 | 5.40 | 0.08 | 4.50 | 0.02 | 0.83 | 0.85 | 56:1 |
|  |  | 5.00 | 5.40 | 0.08 | 2.03 | 0.02 | 0.38 | 0.39 | 25:1 |
|  |  | 5.00 | 5.40 | 0.04 | 4.50 | 0.01 | 0.83 | 0.84 | 113:1 |
|  |  | 1.25 | 2.70 | 0.63 | 0.50 | 0.50 | 0.19 | 0.69 | 1:1.3 |
|  |  | 1.25 | 2.70 | 0.63 | 0.26 | 0.50 | 0.10 | 0.60 | 1:2.4 |
|  |  | 1.25 | 2.70 | 0.63 | 0.13 | 0.50 | 0.05 | 0.55 | 1:4.9 |
|  |  | 1.25 | 2.70 | 0.31 | 0.50 | 0.25 | 0.19 | 0.43 | 1.6:1 |
|  |  | 1.25 | 2.70 | 0.31 | 0.26 | 0.25 | 0.10 | 0.34 | 1:1.2 |
|  |  | 1.25 | 2.70 | 0.18 | 0.50 | 0.14 | 0.19 | 0.33 | 2.8:1 |
|  |  | 1.25 | 2.70 | 0.04 | 0.50 | 0.03 | 0.19 | 0.22 | 13:1 |
|  |  | 1.25 | 2.70 | 0.02 | 0.50 | 0.02 | 0.19 | 0.20 | 25:1 |
|  |  | 0.31 | 2.70 | 0.08 | 1.35 | 0.26 | 0.50 | 0.76 | 17:1 |

Biocide concentrations for stabilized hypochlorite and bromide are reported as ppm total oxidant and CMIT/MIT concentrations are reported as ppm active ingredient.

Table 1 cont. CMIT/MIT(A) and Stabilized Hypochlorite and Bromide (B)

| Day | Expt | A alone | B alone | a comb. | b comb. | a/A | b/B | SI | Ratio of Biocide b:a |
|---|---|---|---|---|---|---|---|---|---|
| 5 | MIC 1 | 0.31 | 2.70 | 0.08 | 1.35 | 0.26 | 0.50 | 0.76 | 17:1 |
|  |  | 0.31 | 2.70 | 0.04 | 1.35 | 0.13 | 0.50 | 0.63 | 34:1 |
|  |  | 1.25 | 2.03 | 0.63 | 0.51 | 0.50 | 0.25 | 0.76 | 1:1.2 |
|  |  | 1.25 | 2.03 | 0.31 | 1.01 | 0.25 | 0.50 | 0.75 | 3.3:1 |
|  |  | 1.25 | 2.70 | 0.63 | 0.51 | 0.50 | 0.19 | 0.69 | 1:1.2 |
|  |  | 1.25 | 2.70 | 0.63 | 0.51 | 0.50 | 0.19 | 0.69 | 1:1.2 |
|  |  | 1.25 | 2.70 | 0.31 | 0.51 | 0.25 | 0.19 | 0.44 | 1.7:1 |
|  |  | 1.25 | 2.70 | 0.18 | 0.51 | 0.14 | 0.19 | 0.33 | 2.8:1 |
|  |  | 1.25 | 2.70 | 0.04 | 0.51 | 0.03 | 0.19 | 0.22 | 13:1 |
| 5 | MIC 2 | 1.25 | 2.70 | 0.63 | 0.51 | 0.50 | 0.19 | 0.69 | 1:1.2 |
|  |  | 1.25 | 2.70 | 0.63 | 0.51 | 0.50 | 0.19 | 0.69 | 1:1.2 |
|  |  | 1.25 | 2.70 | 0.63 | 0.51 | 0.50 | 0.19 | 0.69 | 1:1.2 |
|  |  | 1.25 | 2.70 | 0.31 | 0.51 | 0.25 | 0.19 | 0.44 | 1.7:1 |
|  |  | 1.25 | 2.70 | 0.18 | 0.51 | 0.14 | 0.19 | 0.33 | 2.8:1 |
|  |  | 1.25 | 2.70 | 0.04 | 0.51 | 0.03 | 0.19 | 0.22 | 13:1 |
|  |  | 1.25 | 2.70 | 0.02 | 0.51 | 0.02 | 0.19 | 0.20 | 26:1 |
|  |  | 0.31 | 2.70 | 0.08 | 1.35 | 0.26 | 0.50 | 0.76 | 17:1 |
|  |  | 1.25 | 2.03 | 0.63 | 0.51 | 0.50 | 0.25 | 0.76 | 1:1.2 |
| 5 | MBC 1 | 0.31 | 2.70 | 0.08 | 1.35 | 0.26 | 0.50 | 0.76 | 17:1 |
|  |  | 0.31 | 2.70 | 0.04 | 1.35 | 0.13 | 0.50 | 0.63 | 34:1 |
|  |  | 1.25 | 2.03 | 0.63 | 0.51 | 0.50 | 0.25 | 0.76 | 1:1.2 |
|  |  | 1.25 | 2.03 | 0.31 | 1.01 | 0.25 | 0.50 | 0.75 | 3.3:1 |
| 5 | MBC 2 | 0.31 | 2.70 | 0.08 | 1.35 | 0.26 | 0.50 | 0.76 | 17:1 |
|  |  | 1.25 | 2.70 | 0.63 | 0.50 | 0.50 | 0.19 | 0.69 | 1:1.3 |
|  |  | 1.25 | 2.70 | 0.63 | 0.26 | 0.50 | 0.10 | 0.60 | 1:2.4 |
|  |  | 1.25 | 2.70 | 0.63 | 0.13 | 0.50 | 0.05 | 0.55 | 1:4.9 |
|  |  | 1.25 | 2.70 | 0.31 | 0.50 | 0.25 | 0.19 | 0.43 | 1.6:1 |
|  |  | 1.25 | 2.70 | 0.31 | 0.26 | 0.25 | 0.10 | 0.34 | 1:1.2 |
|  |  | 1.25 | 2.70 | 0.18 | 0.50 | 0.14 | 0.19 | 0.33 | 2.8:1 |
|  |  | 1.25 | 2.70 | 0.04 | 0.50 | 0.03 | 0.19 | 0.22 | 13:1 |
|  |  | 1.25 | 2.70 | 0.02 | 0.50 | 0.02 | 0.19 | 0.20 | 25:1 |

Biocide concentrations for stabilized hypochlorite and bromide are reported as ppm total oxidant and CMIT/MIT concentrations are reported as ppm active ingredient.

**Table 2. Combination of DCOIT (A) and Stabilized Hypochlorite and Bromide (B)**

| Day | Expt | A alone | B alone | a comb. | b comb. | a/A | b/B | SI | Ratio of Biocide b:a |
|---|---|---|---|---|---|---|---|---|---|
| 1 | MIC 1 | 2.50 | 0.50 | 1.25 | 0.07 | 0.50 | 0.14 | 0.64 | 1:18 |
| | | 2.50 | 0.50 | 1.25 | 0.13 | 0.50 | 0.26 | 0.76 | 1:9.6 |
| | | 2.50 | 0.50 | 0.63 | 0.26 | 0.25 | 0.52 | 0.77 | 1:2.4 |
| | | 2.50 | 0.50 | 0.03 | 0.26 | 0.01 | 0.52 | 0.53 | 8.4:1 |
| | | 2.50 | 2.03 | 1.25 | 0.51 | 0.50 | 0.25 | 0.75 | 1:2.5 |
| | | 2.50 | 2.03 | 0.63 | 0.51 | 0.25 | 0.25 | 0.50 | 1:1.2 |
| | | 2.50 | 2.03 | 0.63 | 1.01 | 0.25 | 0.50 | 0.75 | 1.6:1 |
| | | 2.50 | 2.03 | 0.31 | 0.51 | 0.12 | 0.25 | 0.38 | 1.7:1 |
| 1 | MIC 2 | 2.50 | 0.50 | 1.25 | 0.07 | 0.50 | 0.14 | 0.64 | 1:18 |
| | | 2.50 | 0.50 | 1.25 | 0.13 | 0.50 | 0.26 | 0.76 | 1:9.6 |
| | | 2.50 | 0.50 | 0.63 | 0.26 | 0.25 | 0.52 | 0.77 | 1:2.4 |
| | | 2.50 | 0.50 | 0.31 | 0.26 | 0.12 | 0.52 | 0.64 | 1:1.2 |
| | | 2.50 | 2.03 | 1.25 | 0.51 | 0.50 | 0.25 | 0.75 | 1:2.5 |
| | | 2.50 | 2.03 | 0.63 | 1.01 | 0.25 | 0.50 | 0.75 | 1.6:1 |
| | | 2.50 | 2.03 | 0.63 | 0.51 | 0.25 | 0.25 | 0.50 | 1:1.2 |
| | | 2.50 | 2.03 | 0.31 | 1.01 | 0.12 | 0.50 | 0.62 | 3.3:1 |
| | | 2.50 | 2.03 | 0.31 | 1.01 | 0.12 | 0.50 | 0.62 | 3.3:1 |
| 1 | MBC 1 | 10.00 | 2.70 | 5.00 | 0.50 | 0.50 | 0.19 | 0.69 | 1:10 |
| | | 10.00 | 2.70 | 2.50 | 0.50 | 0.25 | 0.19 | 0.44 | 1:5.0 |
| 1 | MBC 2 | 10.00 | 3.30 | 5.00 | 0.50 | 0.50 | 0.15 | 0.65 | 1:10 |
| | | 10.00 | 3.30 | 5.00 | 0.50 | 0.50 | 0.15 | 0.65 | 1:10 |
| | | 10.00 | 3.30 | 2.50 | 0.50 | 0.25 | 0.15 | 0.40 | 1:5.0 |
| | | 10.00 | 3.30 | 2.50 | 0.50 | 0.25 | 0.15 | 0.40 | 1:5.1 |
| | | 10.00 | 3.30 | 1.25 | 0.50 | 0.13 | 0.15 | 0.28 | 1:2.5 |
| | | 10.00 | 3.30 | 1.25 | 0.50 | 0.13 | 0.15 | 0.28 | 1:2.6 |
| | | 10.00 | 3.30 | 0.63 | 0.50 | 0.06 | 0.15 | 0.21 | 1:1.3 |
| | | 10.00 | 3.30 | 0.31 | 0.50 | 0.03 | 0.15 | 0.18 | 1.6:1 |
| | | 10.00 | 3.30 | 0.18 | 0.50 | 0.02 | 0.15 | 0.17 | 2.8:1 |

Biocide concentrations for stabilized hypochlorite and bromide are reported as ppm total oxidant and DCOIT concentrations are reported as ppm active ingredient.

**Table 2 cont. DCOIT (A) and Stabilized Hypochlorite and Bromide (B)**

| Day | Expt | A alone | B alone | a comb. | b comb. | a/A | b/B | SI | Ratio of Biocide b:a |
|-----|------|---------|---------|---------|---------|-----|-----|-----|----------------------|
| 3 | MIC 1 | 5.00 | 3.30 | 2.50 | 0.07 | 0.50 | 0.02 | 0.52 | 1:36 |
|  |  | 5.00 | 3.30 | 2.50 | 0.13 | 0.50 | 0.04 | 0.54 | 1:19 |
|  |  | 5.00 | 3.30 | 2.50 | 0.26 | 0.50 | 0.08 | 0.58 | 1:9.6 |
|  |  | 5.00 | 3.30 | 2.50 | 0.50 | 0.50 | 0.15 | 0.65 | 1:5.0 |
|  |  | 5.00 | 3.30 | 0.63 | 2.70 | 0.13 | 0.82 | 0.94 | 4.3:1 |
|  |  | 5.00 | 3.30 | 0.31 | 2.70 | 0.06 | 0.82 | 0.88 | 8.7:1 |
|  |  | 5.00 | 3.30 | 0.08 | 2.70 | 0.02 | 0.82 | 0.83 | 34:1 |
|  |  | 5.00 | 3.30 | 0.04 | 2.70 | 0.01 | 0.82 | 0.83 | 68:1 |
|  |  | 10.00 | 4.05 | 0.51 | 2.70 | 0.05 | 0.67 | 0.72 | 5.3:1 |
|  |  | 10.00 | 4.05 | 1.01 | 2.70 | 0.10 | 0.67 | 0.77 | 2.7:1 |
|  |  | 10.00 | 4.05 | 2.03 | 2.70 | 0.20 | 0.67 | 0.87 | 1.3:1 |
| 3 | MIC 2 | 10.00 | 3.30 | 5.00 | 0.07 | 0.50 | 0.02 | 0.52 | 1:71 |
|  |  | 10.00 | 3.30 | 5.00 | 0.26 | 0.50 | 0.08 | 0.58 | 1:19 |
|  |  | 10.00 | 3.30 | 5.00 | 0.50 | 0.50 | 0.15 | 0.65 | 1:10 |
|  |  | 10.00 | 3.30 | 2.50 | 0.13 | 0.25 | 0.04 | 0.29 | 1:19 |
|  |  | 10.00 | 3.30 | 2.50 | 0.26 | 0.25 | 0.08 | 0.33 | 1:20 |
|  |  | 10.00 | 3.30 | 2.50 | 0.50 | 0.25 | 0.15 | 0.40 | 1:5.0 |
|  |  | 10.00 | 3.30 | 1.25 | 0.50 | 0.13 | 0.15 | 0.28 | 1:2.5 |
|  |  | 10.00 | 3.30 | 1.25 | 2.70 | 0.13 | 0.82 | 0.94 | 2.2:1 |
|  |  | 10.00 | 3.30 | 0.63 | 2.70 | 0.06 | 0.82 | 0.88 | 4.3:1 |
|  |  | 10.00 | 3.30 | 0.31 | 2.70 | 0.03 | 0.82 | 0.85 | 8.7:1 |
|  |  | 10.00 | 3.30 | 0.18 | 2.70 | 0.02 | 0.82 | 0.84 | 15:1 |
|  |  | 10.00 | 3.30 | 0.04 | 2.70 | 0.00 | 0.82 | 0.82 | 68:1 |
|  |  | 5.00 | 4.05 | 0.51 | 2.70 | 0.10 | 0.67 | 0.77 | 5.3:1 |
|  |  | 5.00 | 4.05 | 1.01 | 2.70 | 0.20 | 0.67 | 0.87 | 2.7:1 |
|  |  | 5.00 | 4.05 | 2.03 | 2.70 | 0.41 | 0.67 | 1.07 | 1.3:1 |
| 5 | MIC 1 | 5.00 | 3.30 | 0.63 | 2.70 | 0.13 | 0.82 | 0.94 | 4.3:1 |
|  |  | 5.00 | 3.30 | 0.31 | 2.70 | 0.06 | 0.82 | 0.88 | 8.7:1 |
|  |  | 5.00 | 3.30 | 0.08 | 2.70 | 0.02 | 0.82 | 0.83 | 34:1 |
| 5 | MIC 2 | 10.00 | 3.30 | 1.25 | 2.70 | 0.13 | 0.82 | 0.94 | 2.2:1 |
|  |  | 10.00 | 3.30 | 0.63 | 2.70 | 0.06 | 0.82 | 0.88 | 4.3:1 |
|  |  | 10.00 | 3.30 | 0.31 | 2.70 | 0.03 | 0.82 | 0.85 | 8.7:1 |
|  |  | 10.00 | 3.30 | 0.18 | 2.70 | 0.02 | 0.82 | 0.84 | 15:1 |

Biocide concentrations for stabilized hypochlorite and bromide are reported as ppm total oxidant and DCOIT concentrations are reported as ppm active ingredient.

**Table 3. Combination of BIT (A) and Stabilized Hypochlorite and Bromide (B)**

| Day | Expt | A alone | B alone | a comb. | b comb. | a/A | b/B | SI | Ratio of Biocide b:a |
|-----|------|---------|---------|---------|---------|------|------|------|---------------------|
| 1 | MIC 1 | 25.00 | 4.00 | 12.50 | 0.07 | 0.50 | 0.02 | 0.52 | 1:179 |
|  |  | 25.00 | 4.00 | 12.50 | 0.13 | 0.50 | 0.03 | 0.53 | 1:96 |
|  |  | 25.00 | 4.00 | 12.50 | 0.26 | 0.50 | 0.07 | 0.57 | 1:48 |
|  |  | 25.00 | 4.00 | 12.50 | 0.50 | 0.50 | 0.13 | 0.63 | 1:25 |
|  |  | 25.00 | 4.00 | 3.13 | 3.30 | 0.13 | 0.83 | 0.95 | 1.1:1 |
|  |  | 25.00 | 4.00 | 1.56 | 2.70 | 0.06 | 0.68 | 0.74 | 1.7:1 |
|  |  | 25.00 | 4.00 | 1.56 | 3.30 | 0.06 | 0.83 | 0.89 | 2.1:1 |
|  |  | 25.00 | 4.00 | 0.78 | 2.70 | 0.03 | 0.68 | 0.71 | 3.6:1 |
|  |  | 25.00 | 4.00 | 0.78 | 3.30 | 0.03 | 0.83 | 0.86 | 4.2:1 |
|  |  | 25.00 | 4.00 | 0.39 | 0.50 | 0.02 | 0.13 | 0.14 | 1.3:1 |
|  |  | 25.00 | 4.00 | 0.39 | 2.70 | 0.02 | 0.68 | 0.69 | 6.9:1 |
|  |  | 25.00 | 4.00 | 0.39 | 3.30 | 0.02 | 0.83 | 0.84 | 8.5:1 |
|  |  | 25.00 | 4.00 | 0.19 | 0.50 | 0.01 | 0.13 | 0.13 | 2.6:1 |
|  |  | 25.00 | 4.00 | 0.19 | 2.70 | 0.01 | 0.68 | 0.68 | 14:1 |
|  |  | 25.00 | 4.00 | 0.19 | 3.30 | 0.01 | 0.83 | 0.83 | 17:1 |
|  |  | 25.00 | 4.00 | 0.10 | 0.50 | 0.00 | 0.13 | 0.13 | 5.0:1 |
|  |  | 25.00 | 4.00 | 0.10 | 2.70 | 0.00 | 0.68 | 0.68 | 27:1 |
|  |  | 25.00 | 4.00 | 0.10 | 3.30 | 0.00 | 0.83 | 0.83 | 33:1 |
| 1 | MIC 2 | 50.00 | 0.50 | 25.00 | 0.07 | 0.50 | 0.14 | 0.64 | 1:357 |
|  |  | 50.00 | 0.50 | 25.00 | 0.13 | 0.50 | 0.26 | 0.76 | 1:192 |
|  |  | 50.00 | 0.50 | 12.50 | 0.13 | 0.25 | 0.26 | 0.51 | 1:96 |
|  |  | 50.00 | 0.50 | 12.50 | 0.26 | 0.25 | 0.52 | 0.77 | 1:96 |
| 1 | MBC 2 | 100.00 | 4.05 | 50.00 | 1.01 | 0.50 | 0.25 | 0.75 | 1:50 |
| 3 | MIC 2 | 100.00 | 3.30 | 50.00 | 0.26 | 0.50 | 0.08 | 0.58 | 1:192 |
|  |  | 100.00 | 3.30 | 50.00 | 0.50 | 0.50 | 0.15 | 0.65 | 1:100 |

Biocide concentrations for stabilized hypochlorite and bromide are reported as ppm total oxidant and BIT concentrations are reported as ppm active ingredient.

**Table 4. Combination of PHMB (A) and Stabilized Hypochlorite and Bromide (B)**

| Day | Expt | A alone | B alone | a comb. | b comb. | a/A | b/B | SI | Ratio of Biocide b:a |
|-----|------|---------|---------|---------|---------|------|------|------|---------------------|
| 1 | MIC 1 | 0.39 | 0.50 | 0.19 | 0.13 | 0.49 | 0.26 | 0.75 | 1:1.5 |
|  |  | 0.39 | 0.50 | 0.10 | 0.26 | 0.26 | 0.52 | 0.78 | 2.6:1 |
|  |  | 0.78 | 2.03 | 0.39 | 0.51 | 0.50 | 0.25 | 0.75 | 1.3:1 |
|  |  | 0.78 | 2.03 | 0.19 | 0.51 | 0.24 | 0.25 | 0.49 | 2.7:1 |
|  |  | 0.78 | 2.03 | 0.19 | 1.01 | 0.24 | 0.50 | 0.74 | 5.3:1 |
| 1 | MIC 2 | 0.39 | 0.50 | 0.19 | 0.13 | 0.49 | 0.26 | 0.75 | 1:1.5 |
|  |  | 0.39 | 2.03 | 0.19 | 0.51 | 0.49 | 0.25 | 0.74 | 2.7:1 |

Table continued

| Day | Expt | A alone | B alone | a comb. | b comb. | a/A | b/B | SI | Ratio of Biocide b:a |
|-----|------|---------|---------|---------|---------|------|------|------|----------------------|
| 1 | MBC 1 | 1.56 | 4.05 | 0.78 | 1.01 | 0.50 | 0.25 | 0.75 | 1.4:1 |
|   |       | 3.13 | 2.70 | 1.56 | 0.13 | 0.50 | 0.05 | 0.55 | 1:12 |
|   |       | 3.13 | 2.70 | 1.56 | 0.26 | 0.50 | 0.10 | 0.59 | 1:6.0 |
|   |       | 3.13 | 2.70 | 1.56 | 0.50 | 0.50 | 0.19 | 0.68 | 1:3.1 |
|   |       | 3.13 | 2.70 | 0.78 | 0.26 | 0.25 | 0.10 | 0.35 | 1:3.1 |
| 1 | MBC 2 | 1.56 | 4.05 | 0.78 | 1.01 | 0.50 | 0.25 | 0.75 | 1.3:1 |
|   |       | 6.25 | 4.00 | 3.13 | 0.07 | 0.50 | 0.02 | 0.52 | 1:45 |
|   |       | 6.25 | 4.00 | 3.13 | 0.13 | 0.50 | 0.03 | 0.53 | 1:24 |
|   |       | 6.25 | 4.00 | 3.13 | 0.26 | 0.50 | 0.07 | 0.57 | 1:12 |
|   |       | 6.25 | 4.00 | 3.13 | 0.50 | 0.50 | 0.13 | 0.63 | 1:6.3 |
|   |       | 6.25 | 4.00 | 1.56 | 0.07 | 0.25 | 0.02 | 0.27 | 1:22 |
|   |       | 6.25 | 4.00 | 1.56 | 0.13 | 0.25 | 0.03 | 0.28 | 1:12 |
|   |       | 6.25 | 4.00 | 1.56 | 0.26 | 0.25 | 0.07 | 0.31 | 1:6.0 |
|   |       | 6.25 | 4.00 | 1.56 | 0.50 | 0.25 | 0.13 | 0.37 | 1:3.1 |
|   |       | 6.25 | 4.00 | 1.56 | 2.70 | 0.25 | 0.68 | 0.92 | 1.7:1 |
|   |       | 6.25 | 4.00 | 0.78 | 0.26 | 0.12 | 0.07 | 0.19 | 1:3.0 |
|   |       | 6.25 | 4.00 | 0.78 | 2.70 | 0.12 | 0.68 | 0.80 | 3.5:1 |
|   |       | 6.25 | 4.00 | 0.78 | 3.30 | 0.12 | 0.83 | 0.95 | 4.2:1 |
|   |       | 6.25 | 4.00 | 0.39 | 2.70 | 0.06 | 0.68 | 0.74 | 6.9:1 |
|   |       | 6.25 | 4.00 | 0.39 | 3.30 | 0.06 | 0.83 | 0.89 | 8.5:1 |
|   |       | 6.25 | 4.00 | 0.19 | 2.70 | 0.03 | 0.68 | 0.71 | 14:1 |
|   |       | 6.25 | 4.00 | 0.19 | 3.30 | 0.03 | 0.83 | 0.86 | 17:1 |
|   |       | 6.25 | 4.00 | 0.10 | 2.70 | 0.02 | 0.68 | 0.69 | 27:1 |
|   |       | 6.25 | 4.00 | 0.10 | 3.30 | 0.02 | 0.83 | 0.84 | 33:1 |
|   |       | 6.25 | 4.00 | 0.05 | 2.70 | 0.01 | 0.68 | 0.68 | 54:1 |
|   |       | 6.25 | 4.00 | 0.05 | 3.30 | 0.01 | 0.83 | 0.83 | 66:1 |
|   |       | 6.25 | 4.00 | 0.02 | 2.70 | 0.00 | 0.68 | 0.68 | 113:1 |
|   |       | 6.25 | 4.00 | 0.02 | 3.30 | 0.00 | 0.83 | 0.83 | 134:1 |

Biocide concentrations for stabilized hypochlorite and bromide are reported as ppm total oxidant and PHMB concentrations are reported as ppm active ingredient.

**Table 4 cont. PHMB (A) and Stabilized Hypochlorite and Bromide (B)**

| Day | Expt | A alone | B alone | a comb. | b comb. | a/A | b/B | SI | Ratio of Biocide b:a |
|---|---|---|---|---|---|---|---|---|---|
| 3 | MIC 1 | 3.13 | 3.30 | 1.56 | 0.13 | 0.50 | 0.04 | 0.54 | 1:12 |
| | | 3.13 | 3.30 | 1.56 | 0.26 | 0.50 | 0.08 | 0.58 | 1:6.0 |
| | | 3.13 | 3.30 | 1.56 | 0.50 | 0.50 | 0.15 | 0.65 | 1:3.1 |
| | | 3.13 | 3.30 | 1.56 | 2.70 | 0.50 | 0.82 | 1.32 | 1.7:1 |
| | | 3.13 | 3.30 | 0.78 | 0.26 | 0.25 | 0.08 | 0.33 | 1:3.0 |
| | | 3.13 | 3.30 | 0.78 | 2.70 | 0.25 | 0.82 | 1.07 | 3.5:1 |
| | | 3.13 | 3.30 | 0.39 | 0.50 | 0.12 | 0.15 | 0.28 | 1.3:1 |
| | | 3.13 | 3.30 | 0.39 | 2.70 | 0.12 | 0.82 | 0.94 | 6.9:1 |
| | | 3.13 | 3.30 | 0.19 | 2.70 | 0.06 | 0.82 | 0.88 | 14:1 |
| | | 3.13 | 3.30 | 0.10 | 2.70 | 0.03 | 0.82 | 0.85 | 27:1 |
| | | 3.13 | 3.30 | 0.02 | 2.70 | 0.01 | 0.82 | 0.83 | 113:1 |
| 3 | MIC 2 | 1.56 | 3.30 | 0.78 | 0.26 | 0.50 | 0.08 | 0.58 | 1:3.0 |
| | | 1.56 | 3.30 | 0.78 | 0.50 | 0.50 | 0.15 | 0.65 | 1:5.6 |
| | | 1.56 | 3.30 | 0.78 | 2.70 | 0.50 | 0.82 | 1.32 | 3.5:1 |
| | | 1.56 | 3.30 | 0.39 | 2.70 | 0.25 | 0.82 | 1.07 | 6.9:1 |
| | | 1.56 | 3.30 | 0.19 | 2.70 | 0.12 | 0.82 | 0.94 | 14:1 |
| 3 | MBC 1 | 3.13 | 3.30 | 1.56 | 0.13 | 0.50 | 0.04 | 0.54 | 1:12 |
| | | 3.13 | 3.30 | 1.56 | 0.26 | 0.50 | 0.08 | 0.58 | 1:6.0 |
| | | 3.13 | 3.30 | 1.56 | 0.50 | 0.50 | 0.15 | 0.65 | 1:3.1 |
| | | 3.13 | 3.30 | 0.78 | 0.26 | 0.25 | 0.08 | 0.33 | 1:3.0 |
| | | 3.13 | 3.30 | 0.39 | 2.70 | 0.12 | 0.82 | 0.94 | 6.9:1 |
| | | 3.13 | 3.30 | 0.19 | 2.70 | 0.06 | 0.82 | 0.88 | 14:1 |
| | | 3.13 | 3.30 | 0.10 | 2.70 | 0.03 | 0.82 | 0.85 | 27:1 |
| | | 3.13 | 3.30 | 0.05 | 2.70 | 0.02 | 0.82 | 0.83 | 54:1 |
| 3 | MBC 2 | 1.56 | 3.30 | 0.78 | 0.26 | 0.50 | 0.08 | 0.58 | 1:3.0 |
| | | 1.56 | 3.30 | 0.78 | 0.50 | 0.50 | 0.15 | 0.65 | 1:5.6 |
| | | 1.56 | 3.30 | 0.19 | 2.70 | 0.12 | 0.82 | 0.94 | 14:1 |
| | | 1.56 | 4.05 | 0.19 | 2.03 | 0.12 | 0.50 | 0.62 | 11:1 |
| | | 1.56 | 4.05 | 0.10 | 2.03 | 0.06 | 0.50 | 0.57 | 20:1 |

Biocide concentrations for stabilized hypochlorite and bromide are reported as ppm total oxidant and PHMB concentrations are reported as ppm active ingredient.

**Table 4 cont. PHMB (A) and Stabilized Hypochlorite and Bromide (B)**

| Day | Expt | A alone | B alone | a comb. | b comb. | a/A | b/B | SI | Ratio of Biocide b:a |
|---|---|---|---|---|---|---|---|---|---|
| 5 | MIC 1 | 3.13 | 3.30 | 1.56 | 0.13 | 0.50 | 0.04 | 0.54 | 1:12 |
| | | 3.13 | 3.30 | 1.56 | 0.26 | 0.50 | 0.08 | 0.58 | 1:6.0 |
| | | 3.13 | 3.30 | 1.56 | 0.50 | 0.50 | 0.15 | 0.65 | 1:3.1 |
| | | 3.13 | 3.30 | 0.78 | 0.26 | 0.25 | 0.08 | 0.33 | 1:3.0 |
| | | 3.13 | 3.30 | 0.39 | 2.70 | 0.12 | 0.82 | 0.94 | 6.9:1 |
| | | 3.13 | 3.30 | 0.19 | 2.70 | 0.06 | 0.82 | 0.88 | 14:1 |
| | | 3.13 | 3.30 | 0.10 | 2.70 | 0.03 | 0.82 | 0.85 | 27:1 |
| | | 3.13 | 3.30 | 0.05 | 2.70 | 0.02 | 0.82 | 0.83 | 54:1 |
| | | 1.56 | 4.05 | 0.78 | 1.01 | 0.50 | 0.25 | 0.75 | 1.3:1 |
| 5 | MIC 2 | 1.56 | 3.30 | 0.78 | 0.13 | 0.50 | 0.04 | 0.54 | 1:6.0 |
| | | 1.56 | 3.30 | 0.78 | 0.26 | 0.50 | 0.08 | 0.58 | 1:3.0 |
| | | 1.56 | 3.30 | 0.78 | 0.50 | 0.50 | 0.15 | 0.65 | 1:5.6 |
| | | 1.56 | 3.30 | 0.19 | 2.70 | 0.12 | 0.82 | 0.94 | 14:1 |
| | | 1.56 | 4.05 | 0.39 | 2.03 | 0.25 | 0.50 | 0.75 | 5.2:1 |
| | | 1.56 | 4.05 | 0.19 | 2.03 | 0.12 | 0.50 | 0.62 | 11:1 |
| | | 1.56 | 4.05 | 0.78 | 1.01 | 0.50 | 0.25 | 0.75 | 1.3:1 |

Biocide concentrations for stabilized hypochlorite and bromide are reported as ppm total oxidant and PHMB concentrations are reported as ppm active ingredient.

**Table 5. Combination of ADBAC (A) and Stabilized Hypochlorite and Bromide (B)**

| Day | Expt | A alone | B alone | a comb. | b comb. | a/A | b/B | SI | Ratio of Biocide b:a |
|---|---|---|---|---|---|---|---|---|---|
| 1 | MIC 1 | 12.50 | 0.50 | 0.39 | 0.26 | 0.03 | 0.52 | 0.55 | 1:1.5 |
| | | 12.50 | 0.50 | 0.10 | 0.26 | 0.01 | 0.52 | 0.53 | 2.6:1.0 |
| 3 | MIC 2 | 60.00 | 2.70 | 25.00 | 0.50 | 0.50 | 0.19 | 0.69 | 1:50 |
| | | 50.00 | 2.70 | 25.00 | 0.26 | 0.50 | 0.10 | 0.60 | 1:97 |
| | | 50.00 | 2.70 | 25.00 | 0.13 | 0.50 | 0.05 | 0.55 | 1:192 |
| 3 | MBC 1 | 50.00 | 2.70 | 25.00 | 0.13 | 0.50 | 0.05 | 0.55 | 1:192 |
| 3 | MBC 2 | 50.00 | 4.05 | 25.00 | 1.01 | 0.50 | 0.25 | 0.75 | 1:25 |
| | | 50.00 | 4.05 | 12.50 | 2.03 | 0.25 | 0.50 | 0.75 | 1:6.2 |

Biocide concentrations for stabilized hypochlorite and bromide are reported as ppm total oxidant and ADBAC concentrations are reported as ppm active ingredient.

**Table 6. Combination of Glutaraldehyde (A) and Stabilized Hypochlorite and Bromide (B)**

| Day | Expt | A alone | B alone | a comb. | b comb. | a/A | b/B | SI | Ratio of Biocide b:a |
|---|---|---|---|---|---|---|---|---|---|
| 1 | MIC 1 | 25.00 | 3.30 | 12.50 | 0.26 | 0.50 | 0.08 | 0.58 | 1:48 |
| 1 | MIC 2 | 25.00 | 3.30 | 12.50 | 0.13 | 0.50 | 0.04 | 0.54 | 1:96 |
| | | 25.00 | 3.30 | 12.50 | 0.26 | 0.50 | 0.08 | 0.58 | 1:48 |
| | | 25.00 | 3.30 | 12.50 | 0.50 | 0.50 | 0.15 | 0.65 | 1:25 |
| 1 | MBC 1 | 25.00 | 3.30 | 12.50 | 0.26 | 0.50 | 0.08 | 0.58 | 1:48 |

Table continued

| Day | Expt | A alone | B alone | a comb. | b comb. | a/A | b/B | SI | Ratio of Biocide b:a |
|---|---|---|---|---|---|---|---|---|---|
| 1 | MBC 2 | 25.00 | 3.30 | 12.50 | 0.13 | 0.50 | 0.04 | 0.54 | 1:96 |
| | | 25.00 | 3.30 | 12.50 | 0.26 | 0.50 | 0.08 | 0.58 | 1:48 |
| | | 25.00 | 3.30 | 12.50 | 0.50 | 0.50 | 0.15 | 0.65 | 1:25 |
| 3 | MIC 1 | 25.00 | 3.30 | 12.50 | 0.26 | 0.50 | 0.08 | 0.58 | 1:48 |
| | | 25.00 | 4.05 | 12.50 | 1.01 | 0.50 | 0.25 | 0.75 | 1:12 |
| 3 | MIC 2 | 50.00 | 3.30 | 25.00 | 0.13 | 0.50 | 0.04 | 0.54 | 1:192 |
| | | 50.00 | 3.30 | 25.00 | 0.26 | 0.50 | 0.08 | 0.58 | 1:96 |
| | | 50.00 | 3.30 | 25.00 | 0.50 | 0.50 | 0.15 | 0.65 | 1:50 |
| 3 | MBC 2 | 50.00 | 3.30 | 25.00 | 0.26 | 0.50 | 0.08 | 0.58 | 1:96 |
| 3 | MBC 2 | 50.00 | 3.30 | 25.00 | 0.50 | 0.50 | 0.15 | 0.65 | 1:50 |
| 5 | MIC 1 | 25.00 | 3.30 | 12.50 | 0.26 | 0.50 | 0.08 | 0.58 | 1:48 |
| 5 | MIC 2 | 50.00 | 3.30 | 25.00 | 0.13 | 0.50 | 0.04 | 0.54 | 1:192 |
| | | 50.00 | 3.30 | 25.00 | 0.26 | 0.50 | 0.08 | 0.58 | 1:96 |
| | | 50.00 | 3.30 | 25.00 | 0.50 | 0.50 | 0.15 | 0.65 | 1:50 |

Biocide concentrations for stabilized hypochlorite and bromide are reported as ppm total oxidant and glutaraldehyde concentrations are reported as ppm active ingredient.

**Table 7. Combination of BNPD (A) and Stabilized Hypochlorite and Bromide (B)**

| Day | Expt | A alone | B alone | a comb. | b comb. | a/A | b/B | SI | Ratio of Biocide b:a |
|---|---|---|---|---|---|---|---|---|---|
| 5 | MIC | 1 100.00 | 8.10 | 0.10 | 6.75 | 0.00 | 0.83 | 0.83 | 68:1 |
| | | 3.13 | 8.10 | 1.56 | 2.03 | 0.50 | 0.25 | 0.75 | 1.3:1 |
| | | 3.13 | 8.10 | 0.78 | 2.03 | 0.25 | 0.25 | 0.50 | 2.6:1 |
| | | 3.13 | 8.10 | 0.78 | 4.05 | 0.25 | 0.50 | 0.75 | 5.2:1 |
| | | 3.13 | 8.10 | 0.78 | 5.40 | 0.25 | 0.67 | 0.92 | 6.9:1 |

Biocide concentrations for stabilized hypochlorite and bromide are reported as ppm total oxidant and BNPD concentrations are reported as ppm active ingredient.

**Table 8. Combination of DBNPA (A) and Stabilized Hypochlorite and Bromide (B)**

| Day | Expt | A alone | B alone | a comb. | b comb. | a/A | b/B | SI | Ratio of Biocide b:a |
|---|---|---|---|---|---|---|---|---|---|
| 1 | MIC 1 | 6.25 | 2.03 | 1.56 | 1.01 | 0.25 | 0.50 | 0.75 | 1:5.1 |
| | | 6.25 | 2.03 | 0.78 | 1.01 | 0.12 | 0.50 | 0.62 | 1.3:1 |
| 1 | MIC 2 | 6.25 | 2.03 | 3.13 | 0.51 | 0.50 | 0.25 | 0.75 | 1:6.1 |
| | | 6.25 | 2.03 | 1.56 | 1.01 | 0.25 | 0.50 | 0.75 | 1:5.1 |
| | | 6.25 | 2.03 | 0.78 | 1.01 | 0.12 | 0.50 | 0.62 | 1.3:1 |
| 1 | MBC 1 | 12.50 | 2.03 | 6.25 | 0.51 | 0.50 | 0.25 | 0.75 | 1:12 |
| 5 | MIC 1 | 12.50 | 6.25 | 4.00 | 0.13 | 0.32 | 0.02 | 0.34 | 1:31 |
| | | 6.25 | 2.03 | 3.13 | 0.51 | 0.50 | 0.25 | 0.75 | 1:6.1 |
| 5 | MIC 1 | 12.50 | 6.25 | 4.00 | 0.50 | 0.32 | 0.08 | 0.40 | 1:8.0 |
| | | 12.50 | 6.25 | 4.00 | 2.70 | 0.32 | 0.43 | 0.75 | 1:1.5 |
| | | 6.25 | 2.03 | 3.13 | 0.51 | 0.50 | 0.25 | 0.75 | 1:6.1 |

Table continued

| Day | Expt | A alone | B alone | a comb. | b comb. | a/A | b/B | SI | Ratio of Biocide b:a |
|-----|------|---------|---------|---------|---------|------|------|------|----------------------|
| 5 | MIC 2 | 12.50 | 6.25 | 4.00 | 0.13 | 0.32 | 0.02 | 0.34 | 1:31 |
| | | 12.50 | 6.25 | 4.00 | 0.50 | 0.32 | 0.08 | 0.40 | 1:8.0 |
| | | 12.50 | 6.25 | 4.00 | 2.70 | 0.32 | 0.43 | 0.75 | 1:1.5 |

Biocide concentrations for stabilized hypochlorite and bromide are reported as ppm total oxidant and DBNPA concentrations are reported as ppm active ingredient.

**Table 9. Combination of BCDMH (A) and Stabilized Hypochlorite and Bromide (B)**

| Day | Expt | A alone | B alone | a comb. | b comb. | a/A | b/B | SI | Ratio of Biocide b:a |
|-----|------|---------|---------|---------|---------|------|------|------|----------------------|
| 1 | MIC 1 | 3.13 | 2.03 | 1.56 | 0.51 | 0.50 | 0.25 | 0.75 | 1:3.1 |
| | | 3.13 | 2.03 | 0.78 | 1.01 | 0.25 | 0.50 | 0.75 | 1.3:1 |
| 1 | MIC 2 | 3.13 | 0.50 | 1.56 | 0.07 | 0.50 | 0.14 | 0.64 | 1:22 |
| | | 3.13 | 0.50 | 1.56 | 0.13 | 0.50 | 0.26 | 0.76 | 1:12 |
| | | 3.13 | 0.50 | 1.56 | 0.13 | 0.50 | 0.26 | 0.76 | 1:12 |
| | | 3.13 | 0.50 | 0.78 | 0.13 | 0.25 | 0.26 | 0.51 | 1:6.0 |
| | | 3.13 | 0.50 | 0.78 | 0.26 | 0.25 | 0.52 | 0.77 | 1:3.0 |
| | | 3.13 | 2.03 | 1.56 | 0.51 | 0.50 | 0.25 | 0.75 | 1:3.1 |
| | | 3.13 | 2.03 | 0.78 | 1.01 | 0.25 | 0.50 | 0.75 | 1.3:1 |
| 1 | MBC 1 | 6.25 | 4.05 | 3.13 | 1.01 | 0.50 | 0.25 | 0.75 | 1:3.1 |
| | | 6.25 | 4.05 | 1.56 | 1.01 | 0.25 | 0.25 | 0.50 | 1:1.5 |
| | | 6.25 | 4.05 | 1.56 | 2.03 | 0.25 | 0.50 | 0.75 | 1.3:1 |
| | | 6.25 | 4.05 | 0.78 | 2.03 | 0.12 | 0.50 | 0.63 | 2.6:1 |
| | | 6.25 | 4.05 | 0.39 | 2.03 | 0.06 | 0.50 | 0.56 | 5.2:1 |
| | | 6.25 | 4.05 | 0.19 | 2.03 | 0.03 | 0.50 | 0.53 | 11:1 |
| 1 | MBC 2 | 6.25 | 4.05 | 3.13 | 1.01 | 0.50 | 0.25 | 0.75 | 1:3.1 |
| | | 6.25 | 4.05 | 1.56 | 2.03 | 0.25 | 0.50 | 0.75 | 1.3:1 |
| | | 6.25 | 4.05 | 1.56 | 1.01 | 0.25 | 0.25 | 0.50 | 1:1.5 |
| | | 6.25 | 4.05 | 0.78 | 2.03 | 0.12 | 0.50 | 0.63 | 2.6:1 |
| | | 6.25 | 4.05 | 0.39 | 2.03 | 0.06 | 0.50 | 0.56 | 5.2:1 |
| | | 6.25 | 4.00 | 1.56 | 0.50 | 0.25 | 0.13 | 0.37 | 1:3.1 |
| | | 6.25 | 4.00 | 1.56 | 2.70 | 0.25 | 0.68 | 0.92 | 1.7:1 |
| | | 6.25 | 4.00 | 0.78 | 2.70 | 0.12 | 0.68 | 0.80 | 3.5:1 |
| | | 6.25 | 4.00 | 0.78 | 3.30 | 0.12 | 0.83 | 0.95 | 4.2:1 |
| | | 6.25 | 4.00 | 0.39 | 2.70 | 0.06 | 0.68 | 0.74 | 6.9:1 |
| | | 6.25 | 4.00 | 0.39 | 3.30 | 0.06 | 0.83 | 0.89 | 8.5:1 |
| | | 6.25 | 4.00 | 0.19 | 2.70 | 0.03 | 0.68 | 0.71 | 14:1 |
| | | 6.25 | 4.00 | 0.19 | 3.30 | 0.03 | 0.83 | 0.86 | 17:1 |
| | | 6.25 | 4.00 | 0.10 | 2.70 | 0.02 | 0.68 | 0.69 | 27:1 |
| | | 6.25 | 4.00 | 0.10 | 3.30 | 0.02 | 0.83 | 0.84 | 33:1 |
| | | 6.25 | 4.00 | 0.05 | 3.30 | 0.01 | 0.83 | 0.83 | 66:1 |

Biocide concentrations for the stabilized hypochlorite and bromide biocide and also BCDMH are reported as ppm total oxidant.

**Table 9 cont. BCDMH (A) and Stabilized Hypochlorite and Bromide (B)**

| Day | Expt | A alone | B alone | a comb. | b comb. | a/A | b/B | SI | Ratio of Biocide b:a |
|---|---|---|---|---|---|---|---|---|---|
| 3 | MIC 1 | 6.25 | 4.05 | 3.13 | 0.51 | 0.50 | 0.13 | 0.63 | 1:6.2 |
| | | 6.25 | 4.05 | 3.13 | 1.01 | 0.50 | 0.25 | 0.75 | 1:3.1 |
| | | 6.25 | 4.05 | 1.56 | 1.01 | 0.25 | 0.25 | 0.50 | 1:1.5 |
| | | 6.25 | 4.05 | 1.56 | 2.03 | 0.25 | 0.50 | 0.75 | 1.3:1 |
| | | 6.25 | 4.05 | 0.78 | 2.03 | 0.12 | 0.50 | 0.63 | 2.6:1 |
| | | 6.25 | 4.05 | 0.39 | 2.03 | 0.06 | 0.50 | 0.56 | 5.2:1 |
| | | 6.25 | 4.05 | 0.19 | 2.03 | 0.03 | 0.50 | 0.53 | 11:1 |
| 3 | MIC 2 | 6.25 | 3.30 | 3.13 | 0.50 | 0.50 | 0.15 | 0.65 | 1:6.3 |
| | | 6.25 | 3.30 | 1.56 | 0.36 | 0.25 | 0.11 | 0.36 | 1:4.3 |
| | | 6.25 | 3.30 | 1.56 | 0.50 | 0.25 | 0.15 | 0.40 | 1:3.1 |
| | | 6.25 | 3.30 | 0.78 | 2.70 | 0.12 | 0.82 | 0.94 | 3.5:1 |
| | | 6.25 | 3.30 | 0.39 | 2.70 | 0.06 | 0.82 | 0.88 | 6.9:1 |
| | | 6.25 | 3.30 | 0.19 | 2.70 | 0.03 | 0.82 | 0.85 | 14:1 |
| | | 6.25 | 3.30 | 0.10 | 2.70 | 0.02 | 0.82 | 0.83 | 21:1 |
| | | 6.25 | 3.30 | 0.02 | 2.70 | 0.00 | 0.82 | 0.82 | 113:1 |
| | | 6.25 | 4.05 | 0.51 | 0.51 | 0.08 | 0.13 | 0.21 | 1:1 |
| | | 6.25 | 4.05 | 1.01 | 1.01 | 0.16 | 0.25 | 0.41 | 1:1 |
| | | 6.25 | 4.05 | 2.03 | 2.03 | 0.32 | 0.50 | 0.83 | 1:1 |
| | | 6.25 | 4.05 | 1.01 | 1.01 | 0.16 | 0.25 | 0.41 | 1:1 |
| | | 6.25 | 4.05 | 2.03 | 2.03 | 0.32 | 0.50 | 0.83 | 1:1 |
| | | 6.25 | 4.05 | 2.03 | 2.03 | 0.32 | 0.50 | 0.83 | 1:1 |
| | | 6.25 | 4.05 | 2.03 | 2.03 | 0.32 | 0.50 | 0.83 | 1:1 |
| 3 | MBC 2 | 6.25 | 3.30 | 3.13 | 0.13 | 0.50 | 0.04 | 0.54 | 1:24 |
| | | 6.25 | 3.30 | 3.13 | 0.50 | 0.50 | 0.15 | 0.65 | 1:6.1 |
| | | 6.25 | 3.30 | 1.56 | 0.26 | 0.25 | 0.08 | 0.33 | 1:6 |
| | | 6.25 | 3.30 | 1.56 | 0.50 | 0.25 | 0.15 | 0.40 | 1:3.1 |
| | | 6.25 | 3.30 | 0.78 | 2.70 | 0.12 | 0.82 | 0.94 | 3.5:1 |
| | | 6.25 | 3.30 | 0.39 | 2.70 | 0.06 | 0.82 | 0.88 | 6.9:1 |
| | | 6.25 | 3.30 | 0.10 | 2.70 | 0.02 | 0.82 | 0.83 | 26:1 |
| | | 6.25 | 3.30 | 0.24 | 2.70 | 0.04 | 0.82 | 0.86 | 11:1 |
| | | 6.25 | 4.05 | 1.56 | 1.01 | 0.25 | 0.25 | 0.50 | 1:1.5 |
| | | 6.25 | 4.05 | 1.56 | 2.03 | 0.25 | 0.50 | 0.75 | 1.3:1 |
| | | 6.25 | 4.05 | 0.78 | 2.03 | 0.12 | 0.50 | 0.63 | 2.6:1 |
| | | 6.25 | 4.05 | 0.39 | 2.03 | 0.06 | 0.50 | 0.56 | 5.2:1 |

Biocide concentrations for the stabilized hypochlorite and bromide biocide and also BCDMH are reported as ppm total oxidant.

**Table 9 cont. BCDMH (A) and Stabilized Hypochlorite and Bromide (B)**

| Day | Expt | A alone | B alone | a comb. | b comb. | a/A | b/B | SI | Ratio of Biocide b:a |
|---|---|---|---|---|---|---|---|---|---|
| 5 | MIC 1 | 6.25 | 4.05 | 3.13 | 0.51 | 0.50 | 0.13 | 0.63 | 1:6.1 |
| | | 6.25 | 4.05 | 3.13 | 1.01 | 0.50 | 0.25 | 0.75 | 1:3.1 |
| | | 6.25 | 4.05 | 1.56 | 1.01 | 0.25 | 0.25 | 0.50 | 1:1.5 |
| | | 6.25 | 4.05 | 1.56 | 2.03 | 0.25 | 0.50 | 0.75 | 1.3:1.0 |
| | | 6.25 | 4.05 | 0.78 | 2.03 | 0.12 | 0.50 | 0.63 | 2.6:1.0 |
| | | 6.25 | 4.05 | 0.39 | 2.03 | 0.06 | 0.50 | 0.56 | 5.2:1.0 |
| | | 6.25 | 4.05 | 0.19 | 2.03 | 0.03 | 0.50 | 0.53 | 11:1.0 |
| 5 | MIC 2 | 6.25 | 3.30 | 3.13 | 0.13 | 0.50 | 0.04 | 0.54 | 1:24 |
| | | 6.25 | 3.30 | 3.13 | 0.50 | 0.50 | 0.15 | 0.65 | 1:6.3 |
| | | 6.25 | 3.30 | 1.56 | 0.26 | 0.25 | 0.08 | 0.33 | 1:6.0 |
| | | 6.25 | 3.30 | 1.56 | 0.50 | 0.25 | 0.15 | 0.40 | 1:3.1 |
| | | 6.25 | 3.30 | 0.78 | 2.70 | 0.12 | 0.82 | 0.94 | 3.5:1 |
| | | 6.25 | 3.30 | 0.39 | 2.70 | 0.06 | 0.82 | 0.88 | 6.9:1 |
| | | 6.25 | 3.30 | 0.19 | 2.70 | 0.03 | 0.82 | 0.85 | 14:1 |
| | | 6.25 | 3.30 | 0.10 | 2.70 | 0.02 | 0.82 | 0.83 | 27:1 |
| | | 6.25 | 3.30 | 0.24 | 2.70 | 0.04 | 0.82 | 0.86 | 11:1 |
| | | 6.25 | 4.05 | 2.13 | 0.51 | 0.34 | 0.13 | 0.47 | 1:4.2 |
| | | 6.25 | 4.05 | 2.13 | 1.01 | 0.34 | 0.25 | 0.59 | 1:2.1 |
| | | 6.25 | 4.05 | 2.13 | 2.03 | 0.34 | 0.50 | 0.84 | 1:1.1 |
| | | 6.25 | 4.05 | 1.56 | 1.01 | 0.25 | 0.25 | 0.50 | 1:1.5 |
| | | 6.25 | 4.05 | 1.56 | 2.03 | 0.25 | 0.50 | 0.75 | 1.3:1 |
| | | 6.25 | 4.05 | 0.78 | 2.03 | 0.12 | 0.50 | 0.63 | 2.6:1 |
| | | 6.25 | 4.05 | 0.39 | 2.03 | 0.06 | 0.50 | 0.56 | 5.2:1 |

Biocide concentrations for the stabilized hypochlorite and bromide biocide and also BCDMH are reported as ppm total oxidant.

**Claims**

1. A microbicidal composition comprising:

   (a) a stabilized hypochlorite and bromide composition; and
   (b) at least one microbicide selected from among 2-methyl-4-isothiazolin-3-one, 5-choloro-2-methyl-4-isothiazolin-3-one, 4,5-dichloro-2-N-octyl-4-isothiazolin-3-one, 1,2-benzisothiazolin-3-one, 3-bromo-1-chloro-5,5-dimethylhydantoin, poly(hexamethylenebiguanide hydrochloride), alkyl-dimethyl-benzylammonium chloride, pentane-1,5-dial, 5-bromo-2-nitro-1,3-propane-diol and 2,2-dibromo-3-nitrilopropionamide.

2. The composition of claim 1 in which a weight ratio of the stabilized hypochlorite and bromide composition to said at least one microbicide is from 1:400 to 300:1.

3. The composition of claim 2 in which the stabilized hypochlorite and bromide composition comprises an alkali metal sulfamate.

**4.** The composition of claim 3 in which said at least one microbicide is a mixture of 5-choloro-2-methyl-4-isothiazolin-3-one and 2-methyl-4-isothiazolin-3-one, wherein a weight ratio of 5-choloro-2-methyl-4-isothiazolin-3-one to 2-methyl-4-isothiazolin-3-one is no more than 4:1.

**5.** The composition of claim 4 in which a weight ratio of the stabilized hypochlorite and bromide composition to said mixture of 5-choloro-2-methyl-4-isothiazolin-3-one and 2-methyl-4-isothiazolin-3-one is from 1:5 to 300:1.

**6.** The composition of claim 5 in which the weight ratio of 5-choloro-2-methyl-4-isothiazolin-3-one to 2-methyl-4-isothiazolin-3-one is from 4:1 to 1:1.

**7.** A method for treating water by adding: (a) a stabilized hypochlorite and bromide composition; and (b) at least one microbicide selected from among 2-methyl-4-isothiazolin-3-one, 5-choloro-2-methyl-4-isothiazolin-3-one, 4,5-dichloro-2-N-octyl-4-isothiazolin-3-one, 1,2-benzisothiazolin-3-one, 3-bromo-1-chloro-5,5-dimethylhydantoin, poly(hexamethylenebiguanide hydrochloride), alkyl-dimethyl-benzylammonium chloride, pentane-1,5-dial (glutaraldehyde), 5-bromo-2-nitro-1,3-propane-diol and 2,2-dibromo-3-nitrilopropionamide.

**8.** The method of claim 7 in which a weight ratio of the stabilized hypochlorite and bromide composition to said at least one microbicide is from 1:500 to 300:1.

**9.** The method of claim 8 in which said at least one microbicide is a mixture of 5 -choloro -2-methyl-4 -isothiazolin- 3 -one and 2-methyl-4-isothiazolin-3-one, wherein a weight ratio of 5-choloro-2-methyl-4-isothiazolin-3-one to 2-methyl-4-isothiazolin-3-one is no more than 4:1.

**10.** The method of claim 7 in which the stabilized hypochlorite and bromide composition is added in an amount from 0.05 to 20 ppm and said at least one microbicide is added in an amount from 0.5 to 500 ppm.